# EUROPÄISCHE PATENTANMELDUNG

(11) **EP 4 751 702 A1**
(43) Veröffentlichungstag der Anmeldung: **03.06.2026**
(21) Anmeldenummer: 25212934.1
(22) Anmeldetag: 03.11.2025
(51) Int. Cl.: A61K 8/06, A61K 8/20, A61K 8/34, A61K 8/67, A61K 8/73, A61K 8/92, A61Q 19/00, A61K 36/185, A61K 31/35, A61K 31/04, A61K 31/045, A61K 31/05, A61K 31/355, A61K 33/14, A61P 17/00, A61P 17/06, A61K 9/00, A61K 9/06

(54) **HAUTVERTRÄGLICHES, PFLEGENDES UND SCHÜTZENDES MITTEL ZUM AUFTRAGEN AUF DIE HAUT**

(30) Priorität: 28.11.2024 DE 202024106904 U
(71) Anmelder: Tulichem GmbH, 55116 Mainz (DE); Merlin Apotheke am Hochhaus Ruth Gebhardt und Dr. Frank Ullrich OHG, 53757 Sankt-Augustin (DE)
(72) Erfinder: POMMERSHEIM, Rainer, 55252 Mainz-Kastel (DE); BREUCH, Denis, 55411 Bingen am Rhein (DE); Löwe, Holger, 55276 Oppenheim (DE)
(74) Vertreter: Meissner Bolte Partnerschaft mbB

(57) **Zusammenfassung**

Die Erfindung betrifft ein hautverträgliches, pflegendes und schützendes Mittel zum Auftragen auf die Haut oder zur Behandlung dieser, aufweisend ein oder mehrere Wirksubstanzen, insbesondere aber nicht ausschließlich, pflanzliche Extrakte oder Öle. Erfindungsgemäß ist eine erste Mischungskomponente vorgesehen, welche mindestens Mandelöl, Cetylalkohol, CBD-Isolat und Vitamin E oder Tocopherol enthält und eine zweite Mischungskomponente vorgesehen, welche im gereinigten Wasser gelöstes Meersalz sowie Xanthan und Propylenglykol aufweist, wobei die ersten und zweiten Mischungskomponenten vereinigt und emulgiert sind.

## Beschreibung

Die Erfindung betrifft ein hautverträgliches, pflegendes und schützendes Mittel zum Auftragen auf die Haut oder zur Behandlung dieser, aufweisend ein oder mehrere Wirksubstanzen, insbesondere, aber nicht ausschließlich, pflanzliche Extrakte oder Öle gemäß Oberbegriff des Anspruches 1.

Bekannt sind Produkte, die auf die Haut aufgetragen werden können und bei welchen durch den Auftrag auf die Haut eine pflegende, aber auch eine desinfizierende, keimmindernde Wirkung resultiert. Diesbezügliche Präparate basieren auf den Wirkstoffen Isopropanol, Ethanol oder anderen Alkoholen.

Mittel zur Hautpflege und zur antimikrobiellen Behandlung der Haut sind beispielsweise aus der EP 1 955 591 A1 bekannt.

Bei dem Gebrauchsmuster DE 20 2020 104 615 U1 handelt es sich um ein desinfizierendes, hautverträgliches, pflegendes und rückfettendes Mittel zum Auftragen auf die Haut. Das Mittel weist ein oder mehrere desinfizierende Wirkstoffe auf und ist dadurch gekennzeichnet, dass wasserlösliche Chlorderivate und/oder ein Alkohol in eine Basis-Matrix eingebunden sind und zusätzlich enthaltend ein Stoffgemisch zur Geruchsmaskierung der speziellen desinfizierenden Werkstoffe.

Medizinisch betrachtet versteht man unter einer Creme oder einer Lotion eine Öl-in-Wasser-Emulsion oder Wasser-in-Öl-Emulsion, die Inhaltsstoffe applikationsfähig macht. Cremes sind relativ zu Salben niedrigviskoser, das heißt flüssiger, und ziehen gut in die Haut ein. Bei Cremes finden häufig Emulgatoren oder Emulsionsstabilisatoren Anwendung. Im allgemeinen Sprachgebrauch werden sie zunehmend auch als nicht-medizinische, das heißt rein pflegende Produkte bezeichnet.

Bei derzeit handelsüblichen Produkten, welche auf die Haut aufgetragen werden können, wird vielfach vom Hersteller eine wohltuende Wirkung versprochen. Derartige Produkte beinhalten in der Regel Gemische von pflanzlichen Ölen oder Extrakten, welche eine physiologische Wirkung entfalten. Bekannt wurden auch Präparate, welche Cannabidiole (CBD) als Inhaltsstoffe enthalten.

Als beispielhafte derartige Produkte seien hier die Bioturm Naturkosmetik CBD Salbe, Wobcare CBD Aktiv Creme, Rubaxx Cannabis CBD Gel, Venostasin Gel oder die Beinwell Creme des Vereins der Benediktiner zu Beuron e.V. benannt.

Bei diesen Produkten werden die entsprechenden Wirkstoffe oder eine Wirkstoffkombination nur in einer einzigen Darreichungsform, zum Beispiel als Salbe, Gel oder aber Creme angeboten.

Aus dem Vorgenannten ist es Aufgabe der Erfindung, ein weiterentwickeltes, hautverträgliches, pflegendes und schützendes Mittel zum Auftragen auf die Haut oder zur Behandlung dieser anzugeben, welches neben der anzustrebenden guten Hautverträglichkeit und der wohltuenden Wirkung in ganz unterschiedlichen Darreichungsformen hergestellt und angeboten werden kann, so dass eine schnelle Reaktion auf entsprechende Kundenwünsche erfolgen kann, wobei die Wirksubstanzen und möglicherweise zusätzliche funktionale und ergänzende Zusatzstoffe beibehalten werden können sowie eine besonders gute Verträglichkeit bei einer durch Neurodermitis oder Psoriasis vorgeschädigten Haut gegeben ist.

Die Lösung der Aufgabe der Erfindung erfolgt durch ein hautverträgliches, pflegendes und schützendes Mittel gemäß Anspruch 1, wobei die Unteransprüche mindestens zweckmäßige Ausgestaltungen und Weiterbildungen darstellen.

Demnach kann je nach gewünschter Darreichungs- und Applikationsform eine bevorratete Wirksubstanzen-Ausgangsbasismischung in eine Trägermatrix eingebunden werden. Die Trägermatrix ist wahlweise als Creme, Lotion oder Salbe, als Gelmatrix oder als alkoholische Creme ausgebildet.

Die Creme kann wasser- oder fettbasiert ausgebildet werden.

Bei der alkoholischen Creme handelt es sich um eine Kombination aus einem alkoholischen Gel und einer fettbasierten Creme.

Der Wirksubstanzen-Ausgangsbasismischung können funktionale Zusatzstoffe beigegeben werden.

Derartige funktionale oder ergänzende Stoffe können Duftstoffe, Farbstoffe, Stoffe zur Geruchsmaskierung, Füllstoffe, Verdickungsmittel, Befeuchtungsmittel und/oder Mittel zum Schutz vor ultravioletter Strahlung sein.

Die Trägermatrix kann auf einer handelsüblichen Basiscreme oder hydrophilen Salbe beruhen.

Die Trägermatrix kann aber auch hydrophiles Gel oder ein lipophiles Gel enthalten.

Die Wirksubstanzen-Ausgangsbasismischung enthält beispielsweise Cannabidiol, ätherische Öle und/oder pflanzliche Isolate.

Bei einer Trägermatrix, welche Gel-basiert ist oder die auf einer alkoholischen Creme basiert, kann eine Zusatzmenge von Alkohol beigegeben werden, um eine verbesserte keimmindernde und desinfizierende Wirkung zu erzielen.

Ausgehend von der Wirksubstanzen-Ausgangsbasismischung kann demnach das hautverträgliche, pflegende und schützende Mittel als Creme oder Salbe auf der Grundlage von Fetten oder Ölen, als Gel auf der Basis von Wasser und/oder Alkohol, als alkoholische Creme, welche sowohl einen hohen Fett- oder Ölgehalt aufweist und gleichzeitig einen hohen Alkoholanteil besitzt, angeboten werden.

Das jeweilige Mittel besteht demnach mindestens aus der Trägermatrix, den Wirksubstanzen und ergänzenden Stoffen, die eine funktionale Wirkung aufweisen können und je nach vorgesehener Anwendung ausgewählt werden.

Diese funktionalen oder ergänzenden Zusatzstoffe können Duftstoffe oder Farbstoffe sein, aber auch Stoffe zur Geruchsmaskierung oder Füllstoffe, wobei auch Stoffe wie zum Beispiel Harnstoff umfasst sind, um eine zusätzliche feuchtende Wirkung zu erzielen. Ebenso können UV-Absorber oder UV-Blocker hinzugegeben werden, wenn der Wunsch besteht, das Mittel als UV-Schutz einzusetzen.

Um unterschiedliche Darreichungsformen bereitzustellen, kann unter Beibehaltung der Wirksubstanzen und der ausgewählten funktionalen oder ergänzenden Zusatzstoffe lediglich ein Austausch der Trägermatrix bzw. eine Variation der Trägermatrix erfolgen.

Die jeweiligen Darreichungsformen des Mittels sollen wie folgt erläutert und mithin definiert werden.

Cremes sind im Sinne der Erfindung Mittel auf der Grundlage von Öl und Wasser. Bei wasserbasierten Cremes liegt eine Öl-in-Wasser-Emulsion vor. Bei fettbasierten Cremes handelt es sich um eine Wasser-in-Öl-Emulsion. Eine Öl-in-Wasser-Creme kann eine kühlende Wirkung erreichen.

Bei Salben handelt es sich um fettbasierte Stoffe, welche nach pharmazeutischer Definition im Unterschied zu Cremes kein Wasser enthalten, jedoch je nach Zusammensetzung in der Lage sind, Wasser aufzunehmen.

Ein Gel wird hier als ein feindispergiertes System definiert, welches aus mindestens einer festen und einer flüssigen Phase besteht. Die feste Phase, zum Beispiel ein Polymer, bildet ein schwammartiges, dreidimensionales Netzwerk, dessen Poren durch eine Flüssigkeit, zum Beispiel ein Alkohol und/oder Wasser oder ein Gas ausfüllbar sind. Beide Phasen durchdringen sich dabei vollständig.

Bei alkoholischen Cremes handelt es sich um Formulierungen, die ähnlich einer herkömmlichen Creme Fette und Wasser enthalten, jedoch zusätzlich einen hohen alkoholischen Anteil aufweisen.

Bei handelsüblichen Cremes ist der Alkoholgehalt fertigungstechnisch auf einen relativ geringen Prozentsatz begrenzt. Die hier erfindungsgemäß eingesetzten alkoholischen Cremes sind eine Kombination aus einem alkoholischen Gel mit einer fettbasierten Creme, wobei auch höhere Alkoholgehalte bis 70 % und mehr erreichbar sind, wodurch sich eine verbesserte desinfizierende Wirkung ergibt.

Die Erfindung soll nachstehend anhand eines Ausführungsbeispieles näher erläutert werden.

Das hautverträgliche, pflegende und schützende Mittel besteht aus den folgenden Stoffen oder Komponenten:

### 1. Die Trägermatrix:

In Abhängigkeit der angestrebten Darreichungsform besteht die Trägermatrix aus unterschiedlichen Stoffen, bzw. ist unterschiedlich aufgebaut, und zwar wie folgt:

### a. Trägermatrix als Creme oder Salbe:

Hierfür kann beispielsweise handelsübliche Basiscremes eingesetzt, die für die Zubereitung von Cremes in der Apotheke angeboten werden. Laut Datenblatt des Herstellers haben sie je nach Formulierung nachfolgend genannte Inhaltsstoffe:
Basiscreme DAC: Glycerolmonostearat 60, Cetylalkohol, Mittelkettige Triglyceride (Neutralöl, Miglyol 812), weißes Vaselin, Macrogol-20-glycerolmonostearat., Propylenglycol, gereinigtes Wasser.

Lanolin DAB: bestehend aus Wollwachs, gereinigtem Wasser und nativem Olivenöl.

Hydrophile Basisemulsion DAC: bestehend aus Sorbitanmonostearat, Typ I, Macrogol-8-stearat, Typ I, Glycerol 85%, Mittelkettige Triglyceride, Citronensäure, Kaliumsorbat, gereinigtes Wasser.

Hydrophobe Basiscreme DAC: bestehend aus Triglyceroldiisostearat, Isopropylpalmitat, Paraffinöl, Polyethylen, Kaliumsorbat, Citronensäure, Magnesiumsulfat, Glycerol 85%, gereinigtes Wasser.

Hydrophile Salbe DAB: bestehend aus dickflüssigem Paraffin, Vaselin, emulgierender Cetylstearylalkohol.

### b. Trägermatrix als Gel:

Bei der Gelmatrix kann es sich um ein hydrophiles Gel handeln, beispielsweise bestehend aus Gelbildnern wie Carbomere, Cellulosederivate wie z.B. Hydroxyethylcellulose, Carboxymethylcellulose, Hydroxypropylmethylcellulose oder Methylcellulose, oder Polysaccharide wie z.B. Alginat, Agar, Xanthan, Guar oder Gellan, zusammen mit Propylenglycol, Glycerin, Ethanol, Natriumedetat, Trometamol und/oder gereinigtem Wasser.

Möglich sind ebenfalls lipophile Gele als Matrix, beispielsweise bestehend aus dickflüssigem Paraffin mit Zusatz von Polyethylen oder Neutralöle mit Verdickungsmitteln wie z.B. Zinkstearat, Aluminiumstearat, hochdisperses Siliziumdioxid oder Ethylcellulose.

### c. Trägermatrix als alkoholische Creme:

Hierbei kann als Basis ein Hydrogel dienen, beispielsweise bestehend aus Carbomer, oder einem anderen der in 1b genannten Gebildnern, gereinigtem Wasser und Ethanol in das ein Neutralöl oder eine Emulsion wie Basiscreme gemäß Ziff. 1.a. oder eine andere Creme oder Salbe eingearbeitet wurde.

### 2. Wirksubstanzen:

Als Wirksubstanzen können diverse Stoffe eingesetzt werden, welche auf der Haut eine Wirkung entfalten. Dies können sowohl pflanzliche Extrakte oder Öle sein, als auch synthetische Stoffe.

Beispielhaft zu nennen wäre insbesondere Cannabidiol (CBD) in Kombination z.B. mit ätherischen Ölen des Johanniskrautes und Arnika.

Des Weiteren zu nennen wären CBD-Öle, Extrakte oder Isolate in Kombination mit z.B. Menthol und Campher.

Möglich sind auch Formulierungen ohne Cannabidiol (CBD) wie z.B.: Ätherische Öle, alkoholische Extrakte sowie Isolate u.a. von Anis, Arnika, Cayennepfeffer, Weihrauch, Kümmel, Gewürznelken, Zimt, Eukalyptus, Fenchel, Lavendel, Zitronen, Kamille, Teebaum, Melissen, Arnika, Hanf, Johanniskraut, Fichten, Kiefernnadeln, Rosmarin, Thymian usw. sowie Kombinationen daraus.

### 3. Funktionale und ergänzende Zusatzstoffe:

### a. Farbstoffe:

Farbstoffe können sein β-Carotin, Riboflavin, Karmin, Curcurmin, Azorubin, Patentblau V, Gold, Titandioxid, Eisenoxide, Mica

### b. Duftstoffe:

Dies können ätherische Öle sein wie z.B.: Rosenöl, Lavendelöl usw. sowie Gemische aus diversen ätherischen Ölen

### c. Stoffe zur Geruchsmaskierung:

Die Erfindung setzt auf kommerzielle erhältliche Stoffgemische, die der Geruchsmaskierung dienen. Beispielhaft ist die Zusammensetzung zweier solcher Produkte angegeben, wie sie aus dem Datenblatt des Herstellers hervorgehen:
Inhaltsstoffe Gemisch A: 2,6-Dimethyloct-7-en-2-ol, 3,7-Dimethyloctan-3-ol, 1-(1,2,3,4,5,6,7,8-Octahydro-2,3,8,8-tetramethyl-2naphthyl)ethan-1-one, Benzenepentanol, beta-methyl- ,Isopentylsalicylate , 3-(p-Ethylphenyl)-2,2-dimethylpropionaldehyde, 3,7-Dimethyloctan-3-yl acetate, Benzenepropanol, beta.,3-trimethyl-, 2-Phenylethanol, Anisaldehyde, (E)-4-(2,6,6-Trimethyl1-cyclohexen-1-yl)-3-buten-2-one, Oxacyclohexadecen-2-one, Piperonal, 1,4-Dioxacycloheptadecane-5,17-dione, Pentadecan-15-olide, Hexyl acetate, 3-(o-Ethylphenyl)-2,2-dimethylpropionaldehyde, 1-Methyl-4-(4methylpentyl)cyclohex3-ene-1-carbaldehyde.

### Inhaltsstoffe Gemisch B:

Hexylsalicylate, 3,7-Dimethyloctan-3-ol , 1-(1,2,3,4,5,6,7,8-Octahydro-2,3,8,8-tetramethyl-2-naphthyl)ethan-1-one , 2H-Pyran-4-ol, tetrahydro-4-methyl-2-(2-methylpropyl)- , Benzenepentanol, beta-methyl- ,Benzenepropanol, beta.,3-trimethyl-, Pentadecan-15-olide, 2,6-Dimethyloct-7-en-2-ol, a-Methyl-1,3-benzodioxole-5-propionaldehyde, 4-(4-Methyl-3-pentenyl)cyclohex-3-ene-1-carbaldehyde, (E)-Anethole, 2-Ethyl-4-(2,2,3-trimethyl-3-cyclopenten1-yl)-2-buten-1-ol, 1,2,3,5,6,7-Hexahydro-1,1,2,3,3-pentamethyl-4H-inden-4-one, 3-(p-Ethylphenyl)-2,2-dimethylpropionaldehyde, MUGETANOL, Piperonal, Oxacyclohexadecen-2-one, 4-Methyl-3-decen-5-ol, 3-(o-Ethylphenyl)-2,2-dimethylpropionaldehyde, Allyl cyclohexyloxy)acetate, Methyl 2,4-dihydroxy-3,6-dimethylbenzoate, 2,6-Dimethylhept-5-enal, Octahydro-7-methyl-1,4-methanonaphthalen6(2H)-one, cis-Hex-3-en-1-yl methyl carbonate.

### d. Füllstoffe und/oder Verdickungsmittel:

Glycerin, Propylenglycol, Paraffin, Stärke oder Stärkederivaten, Titandioxid, Zinkoxid; Polyacrylate, Zellulosederivate, Polyvinylpyrrolidone oder Copolymere dieser Verbindungen.

### e. Funktionale Zusatzstoffe:

Wünscht man eine zusätzliche Wirkung, kann man der Formulierung beispielsweise Harnstoff beifügen. Dadurch wirkt das Produkt zusätzlich befeuchtend. Mischt man der Formulierung beispielsweise einen UV-Blocker wie Titanoxid bei, kann man sie zum Sonnenschutz verwenden. Der Lichtschutzfaktor wird durch die Konzentration an Titanoxid gegeben. Im Falle eines Gels oder einer alkoholischen Creme als Trägermatrix kann der Mischung eine zusätzliche Menge eines Alkohols, z.B. Ethanol bis hin zu Anteilen oberhalb 70% beigefügt werden. Dadurch erreicht man eine keimtötende Wirkung.

Die verkaufsfertige Mittel zum Auftragen auf die Haut besteht aus den oben beschriebenen Komponenten in unterschiedlichen Mischverhältnissen. Sie muss in jedem Fall die Komponenten 1 und 2 und gegebenenfalls 3 enthalten.

Das erfindungsgemäße Mittel kann je nach Darreichungsform in entsprechenden Behältnissen oder Verpackungen bereitgestellt und angeboten werden. Hierfür kommen Spender, Dosen, Tuben oder dergleichen zur Anwendung.

Das erfindungsgemäße Mittel ist besonders bevorzugt zur Anwendung als Handcreme vorgesehen und wird in klassischer Weise durch Einreiben auf die Haut aufgetragen, wodurch sich die gewünschte pflegende bzw. auch desinfizierende Wirkung ergibt.

Die erfindungsgemäße Rezeptur besteht aus den in der nachstehenden Tabelle benannten Komponenten und deren Anteilen:

| **Menge** | **Rohstoff** | **Handelsname** | **Hersteller** |
|---|---|---|---|
| 3,00 - 9,00 g | Meersalz | Totes Meer Badesalz | Salthouse |
| 5,00 - 10,00 g | Cetylalkohol | Cetylalkohol | Sala |
| 0,50 - 3,00 g | CBD-Isolat | CBD-Isolat | |
| 15,00 - 40,00 g | Mandelöl | Mandelöl | Aiesi |
| 0,25 - 1,50 g | Xanthan | Xanthan | |
| 0,05 - 0,20 g | Vitamin E Acetat | D-a-Tocopherol | Caelo |
| 20,00 - 40,0 g | Gereingtes Wasser | | |
| 10,00 - 17,00 g | Propylenglykol | Propylenglykol | Sala |

| | | | |
|---|---|---|---|
| ***a)* Bei der Herstellung und Aufbereitung der Creme wird zunächst das Mandelöl vorgelegt und Cetylalkohol, ggfs. Polidocanol, CBD-Isolat und Vitamin E/Tocopherol hinzugefügt. Diese erhaltene Mischung wird dann unter Rühren bei Temperaturen im Bereich zwischen 60°C und 80°C bis zum vollständigen Lösen erwärmt.** ***b)* Die vorgegebene Menge Meersalz wird bei etwa 160°C bis 180°C für mindestens 1h erhitzt.** ***c)* Das Meersalz wird dann in gereinigtem Wasser gelöst und Xanthan sowie Propylenglykol unter Rühren hinzugefügt.** ***d)* Beide vorerwähnten Ansätze werden dann vereinigt und unter Rühren auf einen Bereich zwischen 60°C und 80°C erwärmt und dann für etwa 10 bis 20 Minuten emulgiert.** | | | |

Die vorteilhafte Wirkung der Formulierung gemäß Rezeptur ist insbesondere bei durch Neurodermitis oder Psoriasis geschädigte Haut gegeben.

Der Einsatz der Hauptwirkstoffe der Rezeptur, nämlich CBD und Totes Meersalz tragen zum erfindungsgemäßen Erfolg bei.

CBD liegt in der Rezeptur im Bereich zwischen 0,5% bis 3%, besonders vorteilhat zu 1% vor. Totes Meersalz liegt im Bereich zwischen 3% und 9%, besonders vorteilhaft mit etwa 8% Anteil vor.

## Patentansprüche

1. Hautverträgliches, pflegendes und schützendes Mittel zum Auftragen auf die Haut oder zur Behandlung dieser, aufweisend ein oder mehrere Wirksubstanzen, insbesondere aber nicht ausschließlich, pflanzliche Extrakte oder Öle,
**dadurch gekennzeichnet, dass**
eine erste Mischungskomponente vorgesehen ist, welche mindestens Mandelöl, Cetylalkohol, CBD-Isolat und Vitamin E oder Tocopherol enthält, eine zweite Mischungskomponente vorgesehen ist, welche im gereinigten Wasser gelöstes Meersalz sowie Xanthan und Propylenglykol aufweist, wobei die ersten und zweiten Mischungskomponenten vereinigt und emulgiert sind.

2. Hautverträgliches Mittel nach Anspruch 1,
**dadurch gekennzeichnet, dass**
ein erster Hauptwirkstoff CBD-Isolat und zweiter Hauptwirkstoff Meersalz ist.

3. Hautverträgliches Mittel nach Anspruch 2,
**dadurch gekennzeichnet, dass**
CBD-Isolat im Bereich zwischen 0,5% bis 3% und Meersalz im Bereich von 3% und 9% in der Mischung enthalten ist.

4. Hautverträgliches Mittel nach einem der vorangegangenen Ansprüche, **dadurch gekennzeichnet, dass**
die Mischung 5g bis 10g Cetylalkohol, 15g bis 40g Mandelöl, 0,25g bis 1,5g Xanthan, 0,05g bis 0,20g Vitamin E oder Tocopherol und 10g bis 17g Propylenglykol enthält und die Menge an gereinigtem Wasser im Bereich von 20g bis 40g liegt.
